# EUROPEAN PATENT APPLICATION

(11) **EP 2 085 079 A2**
(43) Date of publication of application: **05.08.2009**
(21) Application number: 07834489.2
(22) Date of filing: 24.09.2007
(51) Int. Cl.: A61K 9/50, A61K 31/426, A61K 47/30

(54) **PROCESS FOR STABILIZING FAMOTIDINE**

(30) Priority: 25.09.2006 MX PA06/01097
(71) Applicant: WORLD-TRADE IMPORT-EXPORT, WTIE, A.G., 6320 Zug (CH)
(72) Inventor: GARCÍA ARMANTA, María Elena, C.P. 45020 Guadalajara, Jalisco (MX); ÁLVAREZ OCHOA, Victor Guillermo, C.P. 45222 Zapopan, Jalisco, México (MX); SANTOS MURILLO, Josefina, C.P. 44600 Zapopan, Jalisco (MX)
(74) Representative: Carvajal y Urquijo, Isabel
(86) International application number: PCT/MX2007/000108
(87) International publication number: WO 2008/039052

(57) **Abstract**

The present invention relates to pharmaceutical industry, and more specifically with product manufacturing pharmaceutical industry from famotidine. The present invention describes a method to stabilize an alkaline pH for a H2 receptor inhibitor such as Famotidine through a microencapsulation process using as film forming materials, wax or polymer nature products, such as microcrystalline wax, Montan wax, ozokerite wax, chitosan or sodium alginate. Advantages from present invention regarding the state-of-the-art procedures is to make possible with its application, a famotidine composition which resists more the composition medium where prepared and the effects that the composition may have in the digestive tract, specially when this substance is included in a composition containing an alkaline antacid.

## Description

### FIELD OF INVENTION

The present invention concerns to pharmaceutical industry, and more specifically with product manufacturing pharmaceutical industry from famotidine. The present invention describes a method to stabilize an alkaline pH for a H2 receptor inhibitor such as Famotidine through a microencapsulation process using wax or polymer nature products as film forming materials, such as microcrystalline wax, montan wax, ozokerite wax, chitosan or sodium alginate.

### BACKGROUND OF INVENTION

Nowadays, many people are complaining or suffering from gastrointestinal problems going from peptic acid disease, gastritis, colitis, up to more severe problems such as gastroduodenal ulcers and digestive system cancer.

One of the main drugs used for treatment of gastritis, and its main symptoms such as heartburn, burning and stomach acidity is Famotidine. Said product has little stability in alkaline pH thus not being compatible its use with most of antacids which tend to have a pH higher than 8.

The present invention provides an alternative method for famotidine stabilization through microencapsulation in order to prepare alkaline pH liquid compositions (by themselves or combined with any antacid, for instance, Magaldrate) containing Famotidine therein.

Drug microencapsulation from a technological point of view, may be defined as a drug coating process under a molecule, solid particle or liquid globule shape with different type materials, to provide micrometric size particles. Resulting product from this technological process is named "microparticles", "microcapsules" or "microspheres" depending on the used microencapsulation system, systems which are differentiated in their morphology and inner structure, although all of them show a particle size which is always lower than 1 mm as a common feature.

Resulting product from microencapsulation has received several names attending its morphology and inner structure, being the micrometric size a common factor. Microspheres are differentiated from microparticles by the spherical shape from the former. It is important to note that microparticles or microspheres may constitute themselves a pharmaceutical form or be conditioned in a secondary pharmaceutical form. In this way, microparticles may be delivered in a way of suspension or included within a capsule or a tablet. Obviously, the end pharmaceutical form will be conditioned by the route of administration from a microencapsulated product. On this regard, it is important to remark that most of microcapsules currently available in market are targeted to an oral route of administration; however, there is a limited but continuously growing number of parenteral (intramuscular or subcutaneous) delivery microcapsules.

### DESCRIPTION OF INVENTION

The present invention is applicable in the field of pharmaceutical industry and is intended to provide a method for Famotidine stabilization through microencapsulation.

The use of microencapsulation in the present invention resides in the alkaline pH protection (through a drug substance waxy coat) which is exposed within a formulation containing an antacid, such as Magaldrate.

A variety of materials which may be used in microencapsulation is gradually increasing as new biomaterials are developed and new microencapsulation applications are envisaged. Generally, those materials capable of being constituted in microparticles are classified in three categories:
FATS, PROTEINS AND POLYMERS.

Carnauba wax, stearyl alcohol, stearic acid, microcrystalline wax, montan wax and ozokerite wax are found within fats.

Microcrystalline wax is an oil-derived product, formed by hydrocarbon complex mixtures, including normal paraffins as well as monocyclic and polycyclic compounds, which are branched molecular structure compounds with chains from C41 to C57. Microcrystalline waxes have a very high average molecular weight (580-700), with length chains between 40 and 50 carbon atoms, resulting in a high viscosity. Microcrystalline wax is odorless and flavorless with a melting point from 54 to 102°C, soluble in organic solvents, insoluble in acidic and higher than 8 pH. Microcrystalline waxes comprise very small and flexible crystals, generating a higher oil affinity (oil is literally "trapped" within the microcrystalline structure and is unable to migrate into surface).

Montan wax, also known as lignite wax, is obtained from lignite extraction, dark brown-colored or white when bleached, water insoluble and organic solvent soluble. Its composition consists of non-glyceride long chain esters (C24-C30) (62-68 weight %), free long chain organic acids (22-26%), alcohols, cetones and hydrocarbons (7-15%) and long chain resins; its melting point is 79-90 °C.

Carnauba wax is a wax derived from carnauba palm leaves (*Copernicia cerifera*). Generally is present as hard yellow-tan flakes. Carnauba wax contains fatty acid esters (80-85%), fatty alcohols (10-15%), hydroxyacids-acids (3-6%) and hydrocarbon (1-3%). Content of sterified fatty diols (close to 20%), hydroxylated fatty acids (close to 6%) and cinnamic acid (close to 10%) is specific for carnauba wax, compounds which are in various size chains being C26 and C32 the prevailing one. It has a melting point from 80 to 88°C and soluble in organic solvents.

Within proteins and polymers we found proteins such as albumin; within polymers and due to their great versatility this is the more used material family in microencapsulation. Within this great family we may distinguish among natural, semi-synthetic and synthetic polymers. Natural polymers are mainly polysaccharides in nature, with animal and vegetal origin; emphasizing alginate, dextran, Arabic gum (acacia gum) and chitosan. Semi-synthetic polymers include cellulose derivatives which are in a wide variety in market with different solubility features; ethylcellulose and cellulose acetobutyrate, for example, are insoluble polymers while cellulose acetophtalate shows a pH dependant solubility. The more remarkable synthetic polymers are acrylic derivatives and polyesters.

Methods for microencapsulation are varied and they may be grouped in the following groups: Coacervation (phase separation), Interfacial Polymerization; solvent extraction/evaporation, spraying and spraying-freezing, air suspension and ionic gelation.

Under a denomination of "coacervation" or phase "separation" a number of microencapsulation techniques are grouped which are based on induction by any polymer desolvation procedure which in turn, is coated in a coacervation droplet form around the drug to be encapsulated. The term "coacervation' was introduced in colloid chemistry by Bungenberg from Jong and Kruyt in 1929, to describe a macromolecular aggregation or liquid phase separation performed within a colloidal system. Two liquid phases are obtained, one colloid-rich (coacervate) and another colloid-poor (supernatant). Coacervation is an intermediate stage between dissolution and precipitate; that is, involves a partial desolvation opposite to an exhaustive desolvation associated with a precipitation process. Any factor inducing polymer desolvation will produce the coacervation phenomenon. Among the coacervation induction procedures, a change in temperature may be remarked, a pH modification and addition of an incompatible "non-solvent", salt or polymer.

The coacervation microencapsulation process generally includes the following steps:
1. Dispersion through suitable stirring of a compound to be encapsulated (liquid or solid particles) in a coating forming polymer(s) solution.
2. Coacervation induction.
3. Deposition (adsorption) of coacervated droplets around the cores to be encapsulated.
4. Coacervated droplet coalescence to form a continuous coating around the cores.
5. Coacervated coating hardening.

The microencapsulation-coacervation procedure by a temperature change involves the use of a polymer which is soluble in an organic solvent at high temperature and insoluble therein at ambient temperature.

Spraying and spraying-freezing microencapsulation methods performed in a single step, show an advantage of outstanding fastness and simplicity, making them very useful for microparticle industrial production. During spraying, the drug substance is dissolved or dispersed in a polymer solution into a suitable solvent and the mixture is powdered into a chamber where hot air (150-200 °C) able to provide the required vaporization temperature to remove the solvent from the coating material is circulated within, whereby the microencapsulated product is obtained. Spraying-freezing procedure is different from the former in that, instead of spraying the dissolved coating forming material, it is subject to a melting process, then the melted mass being powdered (at a sufficiently high temperature) into a chamber where an air cold stream (20 °C) o a previously cooled gas is circulated. Drug substance is added into the melted mass, dissolved or dispersed therein. Used materials to prepare the coating are low melting point products such as waxes, fats and fatty acids which while solids at ambient temperature, they are melt at a relatively low temperature (40-50 °C).

The method by coacervation used in the present invention, includes the following steps for microencapsulation: the material to be microencapsulated, Famotidine, is suspended in a suspension of the film forming material in a suitable suspension agent (continuous phase). Suspension material may be water, non-polar materials, or simeticone; simeticone being preferred in concentrations from 40 to 90%, preferably 70 to 80%, more preferably 75%. The film forming material which may be any of the following group: ethylcellulose, microcrystalline wax, Carnauba wax, Montan wax, alginate, dextran, Arabic gum (acacia gum) and chitosa); the microcrystalline wax is preferably used in concentrations from 5.0-60.0%, preferably from 10 to 20%, more preferably 17%.

Simeticone is heated at a temperature between 70 and 120°C preferably between 80 to 90°C; microcrystalline wax is separately heated at a temperature between 75 and 125°C, preferably between 85 and 95°C then famotidine is added and mixed at a speed between 1 and 1000 rpm: simeticone starts to be stirred at a speed between 1 and 2000 rpm, preferably 1500 rpm and the mixture containing wax and famotidine is added and stirred up to homogenization. Once prior step is performed, the mixture is rapidly cooled at a temperature between 20-35°C, preferably 30°C to obtain microencapsulated particles.

A first alternative method consists of spraying-freezing microencapsulation, wherein the wax is melted at 80°C and famotidine is dispersed; then the melted mass is powdered (at a sufficiently high temperature) into a chamber where an air cold stream (20-30 °C) or a previously cooled gas, such as nitrogen or freons, is circulated. Drug substance is included in the melted mass dispersed therein which finally is resuspended in simeticone.

A last alternative method is fluid bed microparticle coating using waxes as film forming material and final resuspension in simeticone.

The present invention also describes the use of a resulting microparticle suspension to obtain an oral delivery pharmaceutical form for treatment of a peptic acid disease and related symptoms with excessive production of acid in stomach, further contributing to ulcer healing, as well as to prevent the recurrence from symptomatic esophagus erosions or ulcerations and complications shown along disease suffering. Said product would consist of a mixture of drug substances: Magaldrate gel, Simeticone and Famotidine.

The product obtained with above preparation would consist of: Magaldrate, Simeticone and Famotidine in microcapsules. Moreover, the following functional ingredients are contained: a sweetening agent or sweetener which may be sorbitol, fructose, sucralose, aspartame, saccharose, dextrose, saccharine, sucrose, preferably saccharose in a ratio from 5 to 50%, preferably from 10 to 20%, for example 15%. Another functional ingredient thereof shall help to decrease the surface tension and at the same time to help in stabilizing a suspension formed with microcapsules, whereby lecithin, polysorbates, emulsifying waxes, sorbitan monooleate, glyceryl monooleate, poloxamers, preferably poloxamers, mainly Poloxamer 188 in a ratio from 0.01 to 3%, preferably from 0.1 to 1%, for example 0.5%. Likewise, and in order to keep suspended the microcapsules, a thickening or suspension agent will be added such as sodium or calcium carboxymethylcellulose, gums such as acacia gum, xanthan gum or guar gum, hydroxypropylcellulose, hydroxyethylmethylcellulose, hypromellose, preferably hypromellose in a ratio from 0.01 to 3%, preferably from 0.1 to 1%, for example 0.5%. Finally a flavoring agent may be included.

Finally an analytical method is required which allows a separation in formulation components to be quantified, said method includes an acidic neutralization of Magaldrate for a later extraction from famotidine from the microcapsule with a suitable solvent such as chloroform or hexane or by lipase enzymatic action. Simeticone and Magaldrate quantification are also included by conventional analytical techniques.

The present invention provides a method to provide alkaline pH or antacid mixed formulations wherein famotidine is stable since famotidine is well known to suffer degradation by gastric pH. Further is reported within literature, not to deliver famotidine with antacids (by alkaline degradation). With this project, by protecting famotidine from pH degradation with microencapsulation, Famotidine concentration in duodene might be increased since when going intact through the stomach reaches the duodene without any degradation to be then released by lipase enzyme.

### EXAMPLE:

1.0 Kg of simeticone was added to a processor in this process and heated at 86°C, in one of the ancillary tanks 250 g of microcrystalline wax was added and heated up to 89°C until melting, 100 g of famotidine were added once melted and mixed to full mixing at a speed of 300-800 rpm. Once added, vacuum was applied to the processor to draw air and the mixture Famotidine-wax started to be added to simeticone at a stirring speed of 1500 rpm for 3 minutes. Finally, stirring speed was decreased to 30 rpm and the mixture is then cooled at a temperature of 22 °C to quicken the microencapsulation process.

Said mixture was used to prepare an oral suspension which manufacturing process was as follows: 35 L of water were heated at 65° C to which 500 g of Poloxamer 188 were added to dissolution, once dissolved 500 g of hypromellose were added and temperature was decreased from 25°C then to add the prepared microcapsule total suspension. This solution was added to 40 Kg of Magaldrate to be mixed, once mixed flavor was added thereto and 15 Kg of sugar were dissolved in 10 L of water and previously filtered and finally sufficient water was added for 100 L.

The invention has been sufficiently described so that a person with average skill in this matter may reproduce and obtain the results mentioned in the present invention. However, anyone skilled in the art to whom present invention is concerned may perform modifications not described in present application, however, said structures shall be comprised within the scope of the invention when applying these modifications into a determined composition or in the manufacturing process thereof the claimed matter in following claims is required.

## Claims

1. A method of Famotidine stabilization **characterized in that** being carried out in alkaline media with a pH higher than 8 through a microencapsulation process.

2. A microencapsulation method according to claim 1, **characterized in that** the used method is coacervation.

3. A microencapsulation method according to claim 1, **characterized in that** the used method is spraying-freezing.

4. A method of Famotidine stabilization according to claim 1, **characterized in that** the used method is wax coating in fluid bed.

5. A microencapsulation method according to claim 2, **characterized in that** Simeticone is used as suspension agent, and a fat or a polymer as a film forming agent.

6. A microencapsulation method according to claim 5 wherein the film forming agent is a natural, synthetic or semi-synthetic polymer from the following group: sodium alginate, dextran, Arabic gum (acacia gum), chitosan, cellulose derivatives such as ethylcellulose, cellulose acetobutyrate and cellulose acetophtalate, and acrylic derivatives as well as polyesters.

7. A microencapsulation method according to claim 5 wherein the film forming agent is a fat from the following group: carnauba wax, stearyl alcohol, stearic acid, microcrystalline wax, Montan wax and ozokerite wax.

8. A microencapsulation method according to claim 7 wherein the film forming agent is microcrystalline wax in a percentage within the microencapsulation complex from 5.0-60.0%, preferably from 10 to 20%.

9. A microencapsulation method according to claim 7 wherein the film forming agent is microcrystalline wax and being present in a percentage of 17%.

10. A microencapsulation method according to claim 5 wherein the suspension agent is simeticone in a percentage within the microencapsulation complex of 50-90.0%, preferably from 70 to 80%.

11. A microencapsulation method according to claim 2 and 5 wherein the ingredient to be microencapsulated is Famotidine which is present within the microencapsulation complex in a percentage from 1 to 20% preferably from 15 to 18%.

12. A microencapsulation method according to claim 2 and 5 consisting of heating Simeticone at a temperature between 70 and 120°C, preferably between 80 to 90°C; heating separately microcrystalline wax at a temperature between 75 and 125°C, preferably between 85 and 95°C where famotidine is added and is mixed at a speed between 1 and 1000 rpm; then simeticone is stirred at a speed between 1 and 2000 rpm, preferably 1500 rpm and the mixture comprising wax and famotidine is added and homogenized. Once performed the suspension is rapidly cooled at a temperature between 20-35°C, preferably 30°C to obtain microencapsulated particles.

13. The use of a suspension obtained by any of the processes from claims 2, 3, 4 and 12 which together with Magaldrate provides a product for treatment of peptic acid disease and symptoms related with excessive production of acid in stomach.

14. A product formulated according to claim 13 **characterized by** containing a famotidine microcapsule suspension coated with microcrystalline wax in simeticone, in addition to a sweetening agent, a surfactant agent, a suspension agent, and a flavoring agent.

15. A product formulated according to claim 14 wherein the sweetening agent may be sorbitol, fructose, sucralose, aspartame, saccharose, dextrose, saccharine, sucrose, in a ratio from 5 to 50%, preferably from 10 to 20%, for example 15%.

16. A product formulated according to claim 15 wherein the sweetening agent is saccharose.

17. A product formulated according to claim 14 wherein the surfactant agent may be lecithin, polysorbates, emulsifying waxes, sorbitan monooleate, glyceryl monooleate, poloxamers, in ratios from 0.01 to 3%, preferably from 0.1 to 1%, for example 0.5%

18. A product formulated according to claim 17 wherein the surfactant agent is Poloxamer 188.

19. A product formulated according to claim 14 wherein the suspension agent may be sodium or calcium carboxymethylcellulose, gums such as acacia gum, xanthan gum or guar gum, hydroxypropylcellulose, hydroxyethylmethylcellulose, hypromellose in ratios from 0.01 to 3%, preferably from 0.1 to 1%, for example 0.5%.

20. A product formulated according to claim 19 wherein the suspension agent is hypromellose.
